# EUROPÄISCHE PATENTSCHRIFT

(11) **EP 2 403 354 B1**
(45) Veröffentlichungstag und Bekanntmachung des Hinweises auf die Patenterteilung: **26.12.2012**
(21) Anmeldenummer: 10706261.4
(22) Anmeldetag: 04.03.2010
(51) Int. Cl.: A23K 1/16, A23L 1/275, C07C 403/24

(54) **FORMULIERUNG VON ASTAXANTHIN-DERIVATEN UND DEREN VERWENDUNG**
FORMULATION OF ASTAXANTHIN DERIVATIVES AND USE THEREOF
FORMULATION DE DÉRIVÉS D'ASTAXANTHINE ET LEUR UTILISATION

(30) Priorität: 05.03.2009 EP 09154433
(43) Veröffentlichungstag der Anmeldung: 11.01.2012
(73) Patentinhaber: BASF SE, 67056 Ludwigshafen (DE)
(72) Erfinder: FELDTHUSEN JENSEN, Jesper, 55268 Nieder-Olm (DE); KÖPSEL, Christian, 69469 Weinheim (DE); END, Lutz, 68526 Ladenburg (DE); ENGEL, Robert, 67346 Speyer (DE); GOTTSCHALK, Thomas, 68165 Mannheim (DE); BRANDS, Mario, 67063 Ludwigshafen (DE); PELLETIER, Wolf, 76879 Ottersheim (DE)
(86) Internationale Anmeldenummer: PCT/EP2010/052756
(87) Internationale Veröffentlichungsnummer: WO 2010/100226

(56) Entgegenhaltungen:
- EP-A2- 0 845 503
- WO-A1-03/066583
- WO-A1-2006/125591
- WO-A2-2009/027499

## Beschreibung

Die vorliegende Erfindung betrifft Formulierungen von Astaxanthin-Derivaten in für Nahrungsmittel geeigneten Ölen, Wachsen oder Fetten, sowie die Verwendung der Formulierungen zur Herstellung von Futtermitteln. Die Erfindung betrifft auch ein Verfahren zur Herstellung derartiger Formulierungen und ein Verfahren zur Herstellung von Futtermitteln unter Verwendung der Formulierungen.

Astaxanthin (3,3'-Dihydroxy-β,β-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördem deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel.

Bei der Herstellung derartiger Lösungen erweist sich die schlechte Lösungskinetik des Astaxanthins als problematisch.

Die EP 845503 beschreibt flüssige mit Öl mischbare Carotinoid-Zubereitungen in Form einer Wasser-in-Öl-Emulsion, worin die dispergierte wässrige Phase schutzkollidstabilisierte Carotinoid-Partikel in suspendierter Form enthält. Das Verfahren zur Herstellung dieser Zubereitungen ist vergleichsweise aufwendig. Zudem ist der Anteil an Carotinoid vergleichsweise gering.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Dieses Verfahren ist vergleichsweise aufwendig und bedarf größerer Mengen an Schutzkolloiden.

Die WO 2006/125591 beschreibt die Herstellung von öligen Carotinoid-Lösungen, beispielsweise Lösungen des Astaxanthins, wobei man zunächst eine Suspension des Carotinoids in der Ölphase bereitstellt, die Suspension für eine kurze Zeit auf hohe Temperaturen, z. B. im Bereich von 100 bis 230 °C erhitzt, um das Carotinoid in Lösung zu bringen, und die heiße Lösung mit einem kalten Öl vermischt. Auf diese Weise lassen sich nur sehr verdünnte Lösungen des Astaxanthins herstellen.

Die ältere internationale Patentanmeldung PCT/EP2008/061384 beschreibt ein Verfahren zur Herstellung von öligen Lösungen des Astaxantins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxantin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf. Auf diese Weise lassen sich Lösungen derartiger Astaxanthin-Derivate in für Nahrungsmittel geeigneten Ölen herstellen, die eine Konzentration an Astaxanthin-Derivat von bis zu 2 Gew.-% aufweisen und worin das Astaxanthin-Derivat zu mehr als 80 % in der all-trans-Konfiguration vorliegt. Lösungen mit einer höheren Konzentration an Astaxanthin-Derivat sind mit den in PCT/EP2008/061384 gelehrten Verfahren nicht herstellbar.

Es wurde gefunden, dass die in PCT/EP2008/061384 beschriebenen Lösungen bereits bei Konzentration von 1,8 Gew.-% zur Kristallisation des darin enthaltenen Astaxanthin-Derivats neigen. Bei Konzentrationen oberhalb 2 Gew.-% sind sie regelrecht instabil.

Der vorliegenden Erfindung liegt die Aufgabe zu Grunde, geeignete Formulierungen von Astaxanthin oder einem Astaxanthin-Derivat bereitzustellen, die es erlauben, Astaxanthin oder das Astaxanthin-Derivat in ein für Nahrungsmittel geeignetes Öl einzuarbeiten. Insbesondere sollten die Formulierungen einen hohen Gehalt an Astaxanthin bzw. Astaxanthin-Derivaten aufweisen und über einen längeren Zeitraum stabil bleiben.

Es wurde gefunden, dass die im Folgenden definierten Astaxanthin-Verbindungen der Formel I, sowie Astaxanthin-Derivate, welche zu wenigstens 80 Gew.-% aus wenigstens einer Verbindung der Formel I bestehen, stabile Lösungen in für Nahrungsmittel geeigneten Ölen, Fetten und Wachsen und deren Gemischen mit eine Gehalt an Astaxanthin-Derivat von mehr als 2 Gew.-% bilden, wenn das Astaxanthin-Derivat zu weniger als 80 Gew.-%, insbesondere zu höchstens 75 Gew.-% und speziell zu höchsten 70 Gew.-% in der all-trans-Konfiguration vorliegt. Bei den Astaxanthin-Derivaten handelt es sich um Verbindungen der folgenden Formel I 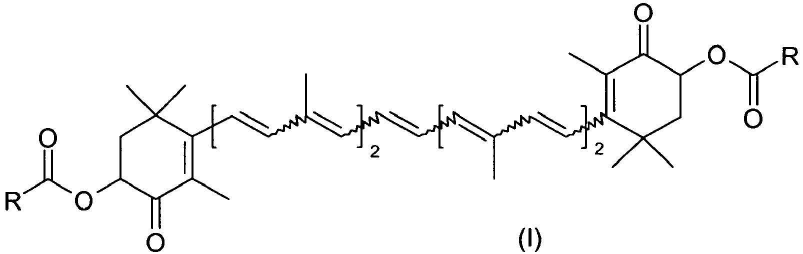 worin R für einen Rest der Formel 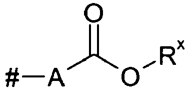 steht, worin # die Anknüpfung an die Carbonylgruppe bedeutet, A für -CH₂CH₂- oder für -CH=CH- steht und R^{x} für C₁-C₄ Alkyl steht; sowie um Astaxanthin-Derivate, die zu wenigstens 80 Gew.-%, insbesondere wenigstens 90 Gew.-% und speziell zu wenigstens 96 Gew.-% aus den Verbindungen der Formel I bestehen.

Derartige Astaxanthin-Derivate und Verfahren zu ihrer Herstellung sind aus der WO 03/066583 A1 bekannt.

Dementsprechend betrifft die vorliegende Erfindung Formulierungen von Astaxanthin-Derivaten in Form einer Lösung des Astaxanthin-Derivats in einem für Nahrungsmittel geeigneten organischen Lösungsmittel, das unter Ölen, Fetten und Wachsen und deren Gemischen ausgewählt ist, mit einem Gehalt an Astaxanthin-Derivat von mehr als 2 Gew.-%, bezogen auf das Gesamtgewicht der Formulierung, wobei das Astaxanthin-Derivat zu wenigstens 80 Gew.-%, insbesondere wenigstens 90 Gew.-% und speziell zu wenigstens 96 Gew.-% aus wenigstens einer Verbindung der Formel I besteht, wobei der Anteil des all-trans-Isomeren (I-trans), bezogen auf die Gesamtmenge der in der Lösung enthaltenen Verbindung der Formel I, weniger als 80 %, insbesondere höchstens 75 Gew.-% und speziell höchsten 70 Gew.-%, insbesondere im Bereich von 40 % bis < 80 %, bevorzugt im Bereich von 45 % bis 75 % und speziell im Bereich von 50 bis 70 % liegt.

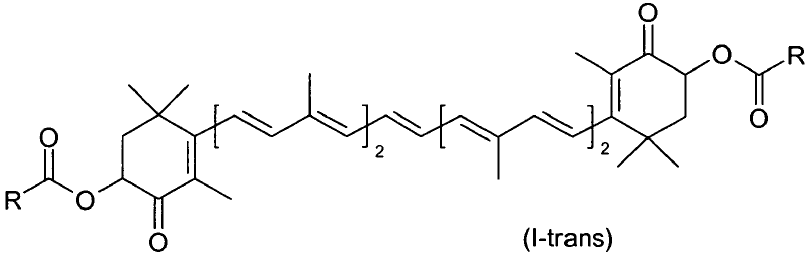

Die Erfindung ist mit einer Reihe von Vorteilen verbunden. Zum einen lassen sich unter Verwendung der erfindungsgemäßen Lösungen in einfacher Weise Lösungen von Astaxanthin-Derivaten in flüssigen, für Nahrungsmittel geeigneten Ölen durch Verdünnen der erfindungsgemäßen Formulierungen mit dem für Nahrungsmittel geeigneten flüssigen Öl herstellen. Außerdem lassen sich unter Verwendung von Astaxanthin-Derivaten, die zu wenigstens 80 Gew.-% aus wenigstens einer Verbindungen der Formel I bestehen, sehr viel einfacher konzentrierte Lösungen in für Nahrungsmittel geeigneten Ölen, Fetten oder Wachsen herstellen als von Astaxanthin selber oder von anderen, davon verschiedener Astaxanthin-Derivate wie Astaxanthindiacetat, Astaxanthindisuccinat oder Astaxanthindipalmitat.

Die erfindungsgemäßen Formulierungen haben neben einer guten Verdünnbarkeit in den für Nahrungsmittel geeigneten Ölen den Vorteil einer verbesserten Handhabbarkeit und Stabilität im Vergleich zu den aus PCT/EP2008/061384 bekannten flüssigen Suspensionen.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form) und die 3S,3'S-Form und die 3R,3'R-Form bilden ein Enantiomerenpaar. Für die Erfindung können sowohl die reinen Enantiomere und Diasteromere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzt, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

In den erfindungsgemäßen Formulierungen weist das Astaxanthin-Derivat üblicherweise eine Reinheit, bezogen auf das in Formel I-trans gezeigten all-trans-Isomer, von 40 bis < 80 Gew.-%, vorzugsweise 45 bis 75 Gew.-% und insbesondere 50 bis 70 Gew.-%, bezogen auf die in der Formulierung enthaltene Verbindung der Formel I auf. Neben dem all-trans-Isomeren enthält das Astaxanthin-Derivat auch Anteile an Astaxanthin-Verbindungen der Formel I, in denen eine oder mehrere der in Formel I dargestellten Doppelbindungen cis-Konfiguration aufweisen. Derartige Verbindungen werden im Folgenden auch als cis-Isomere bezeichnet. Der Anteil an all-trans-Isomer und cis-Isomer in der Formulierung kann mittels Hochdruckflüssigkeitschromatographie (HPLC) ermittelt werden.

Die Gesamtmenge an all-trans-Isomer der Formel I-trans und cis-Isomeren der Formel I beträgt in der Regel wenigstens 80 Gew.-%, häufig wenigstens 90 Gew.-%, insbesondere wenigstens 96 Gew.-%, bezogen auf die Gesamtmenge des in der Formulierung enthaltenen Carotinoid-Derivats. Neben dem all-trans-Isomeren I-trans und den cis-Isomeren kann das Astaxanthin-Derivat auch weitere Carotinoide umfassen, wobei der Anteil dieser Verunreinigungen in der Regel 20 Gew.-%, insbesondere 10 Gew.-%, besonders bevorzugt 4 Gew.-%, bezogen auf die Gesamtmenge des eingesetzten Astaxanthin-Derivats nicht überschreitet. Bei diesen Verunreinigungen handelt es sich in erster Linie um Halbester des Astaxanthins, d. h. um Verbindungen der im Folgenden gezeigten Formel II 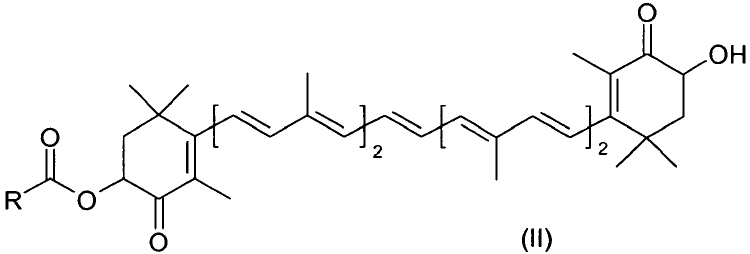 worin R die zuvor genannten Bedeutungen hat bzw. um cis-Isomere davon, sowie um Astaxanthin selber, Adonirubin und/oder Semi-Astacin.

Insbesondere enthält die erfindungsgemäße Formulierung ein Astaxanthin-Derivat, das die an ein für Nahrungsmittel (d. h. für Lebens- und/oder Futtermittel) zugelassenes Astaxanthin gestellten Anforderungen erfüllt, d. h. das weniger als 5 Gew.-% Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.-% und speziell zu wenigstens 96 Gew.-%, bezogen auf die Gesamtmenge an Astaxanthin-Derivat in der Zubereitung, aus einer Astaxanthin-Verbindung der Formel I besteht.

Die erfindungsgemäßen Formulierungen enthalten in der Regel 2,05 bis 5 Gew.-%, insbesondere 2,1 bis 4 Gew.-% und speziell 2,1 bis 3,5 Gew.-%, bezogen auf das Gesamtgewicht der Formulierung, wenigstens eines feinteiligen Astaxanthin-Derivats, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zubereitung, aus einer Astaxanthin-Verbindung der Formel I besteht.

Erfindungsgemäß liegen die in der Zusammensetzung enthaltenen Astaxanthin-Derivate in gelöster Form vor, d. h. wenigstens 98 Gew.-% des in der Formulierung enthaltenen Astaxanthin-Derivats ist molekular-dispers gelöst und kann nicht mehr durch optische Maßnahmen wie Lichtstreuung oder durch Filtration über einen Filter mit einer Porenweite von 0,2 µm als Feststoff detektiert werden.

Erfindungsgemäß handelt es sich bei den in der Formulierung enthaltenen Lösungsmitteln um ein für Nahrungsmittel geeignetes Öl, Fett oder Wachs oder um eine Mischung davon.

Unter einer für Nahrungsmittel zugelassenen Substanz versteht man im Sinne der Erfindung eine für die Tier- und/oder Humanernährung zugelassene Substanz. Der Begriff "Nahrungsmittel" umfasst hier und im Folgenden Nahrungsmittel für die Humanernährung (= Lebensmittel) und Nahrungsmittel für die Tierernährung (= Futtermittel).

Die Begriffe "Öl", "Fett" und Wachs sind hierbei weit zu verstehen und umfassen alle flüssigen und festen organischen Substanzen, die langkettige gesättigte oder ungesättigte Kohlenwasserstoffketten mit in der Regel wenigstens 8 C-Atomen, z. B. 8 bis 30 C-Atomen, aufweisen, die mit Wasser nicht oder nur begrenzt mischbar sind, und die unzersetzt bei Temperaturen von maximal 100 °C schmelzen. Die Verbindungen sind naturgemäß aus Kohlenstoff, Wasserstoff, Sauerstoff und gegebenenfalls Stickstoff aufgebaut und enthalten darüber hinaus keine weiteren Elemente. Der Anteil an Kohlenwasserstoffketten in den Ölen, Wachsen und Fetten liegt in der Regel bei wenigstens 60 Gew.-%, insbesondere wenigstens 70 Gew.-% und speziell wenigstens 80 Gew.-%, bezogen auf das Gesamtgewicht des Lösungsmittels. Im Unterschied zu einem Fett oder Wachs weist ein Öl in der Regel einen Schmelzpunkt unterhalb 40 °C und insbesondere maximal 30 °C auf, wohingegen ein Fett oder Wachs typischerweise einen höheren Schmelzpunkt von in der Regel wenigstens 40 °C aufweist.

Der begriff "fest" im Hinblick auf die erfindungsgemäßen Formulierungen und Lösungsmittel bedeutet, dass sie sich bei Normalbedingungen (298,15 K, 1013 hPa) im festen Aggregatzustand befinden.

Beispiele für Öle, Fette und Wachse sind Fettsäuren, Fettsäureester, Fettalkohole, aliphatische Kohlenwasserstofföle und Wachse, die für Nahrungsmittel geeignet sind.

Die Fettsäuren weisen in der Regel 8 bis 30 C-Atome auf (im Folgenden auch als C₈-C₃₀-Fettsäuren). Die Fettsäuren können gesättigt oder ein oder mehrfach, z. B. 1-, 2- oder 3-fach ungesättigt sein. Geeignet sind auch Fettsäuregemische.

Als Ester der Fettsäuren kommen insbesondere Fettsäureester des Glycerins, z. B. Mono-, Di- und Triglyceride von Fettsäuren und deren Gemische sowie Fettsäureester von Fettalkoholen in Betracht, wobei Fettsäurerest in der Regel 8 bis 30 C-Atome, insbesondere 10 bis 26 C-Atome aufweist und gesättigt oder ein oder mehrfach, z. B. 1-, 2- oder 3-fach ungesättigt sein kann. Der Fettalkoholrest ist in der Regel gesättigt und weist typischerweise 8 bis 30 C-Atome auf (C₈-C₃₀-Fettalkohol). Geeignet sind auch C₃-C₈-Alkylester von Fettsäuren. Die Glyceride von Fettsäuren können natürlichen Ursprungs, semi-synthetisch und vollsynthetisch sein. Beispiele für Glyceride natürlichen Ursprungs sind pflanzliche und tierische Fette und Öle, beispielsweise Pflanzenöle wie Sojabohnen-Öl, Palmöl, Palmkernöl, Sonnenblumenöl, Maiskeimöl, Leinsamenöl, Rapsöl, Distelöl, Weizenkeimöl, Reisöl, Kokosöl, Mandelöl, Aprikosenkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), tierische Öle und Fette wie Fischöle einschließlich Makrelen-, Sprotten-, Thunfisch-, Heilbutt-, Kabeljau- und Lachsöl, Rindertalg, Talgöl, Presstalg und Lanolin. Beispiele für semisynthetische Triglyceride sind Caprylsäure/Caprinsäure-Tricyceride wie die Miglyol-Typen. Beispiele für semisynthetische Fette sind hydrierte Pflanzenöle und hydrierte tierische Öle und Fette.

Als Fettalkohole kommen insbesondere gesättigte aliphatische Alkohole mit 8 bis 30 C-Atome (im Folgenden auch als C₈-C₃₀-Fettsalkohole) in Betracht.

Als aliphatische Kohlenwasserstofföle kommen insbesondere Paraffinöle, hydrierte, flüssige Polyisobutene, Squalane und Squalene in Betracht.

Als Wachse kommen insbesondere natürliche Wachse pflanzlichen oder tierischen Ursprungs wie Bienenwachs, Candelilawachs, Schellack-Wachs, Karitefett und Carnaubawachs, Kohlenwasserstoffwachse, wie Paraffinwachse, Ceresin, Sasolwachse, Ozokerit und Mikrowachse in Betracht.

Bevorzugte Lösungsmittel sind ausgewählt unter
- Mono-, Di- und Triglyceriden von Fettsäuren und deren Gemischen, vorzugsweise von Fettsäuren mit 8 bis 30 C-Atomen, insbesondere mit 10 bis 26 C-Atomen, einschließlich der vorgenannten tierischen Fette und Öle, der vorgenannten Pflanzenöle, der vorgenannten semisynthetischen Triglyceride und der vorgenannten hydrierten Pflanzenöle und der hydrierten tierische Öle und Fette;
- Fettalkoholen mit 8 bis 30 C-Atomen, insbesondere mit 10 bis 26 C-Atomen und
- Estern von C₈-C₃₀-Fettsäuren mit C₈-C₃₀-Fettalkoholen.

Bei den vorgenannten für Nahrungsmittel geeigneten pflanzlichen oder tierischen Ölen und Fetten kann es sich um Raffinate oder um Rohöle bzw. Rohfette handeln, die noch ursprungsspezifische Verunreinigungen wie Proteine, Phosphat, (Erd)alkalimetallsalze und dergleichen in üblichen Mengen enthalten.

Die in den erfindungsgemäßen Zusammensetzungen enthaltenen Öle und Fette können geringe Mengen an emulgiertem oder gelöstem Wasser, vorzugsweise jedoch nicht mehr als 10 Gew.-%, insbesondere nicht mehr als 1 Gew.-%, und speziell nicht mehr als 0,1 Gew.-%, bezogen auf die Gesamtmenge des eingesetzten Öl bzw. Fett, enthalten.

Eine bevorzugte Ausführungsform der Erfindung betrifft feste Formulierungen von Astaxanthin-Derivaten in Form einer festen Lösung des Astaxanthin-Derivats in einem für Nahrungsmittel geeigneten Lösungsmittel. Das Lösungsmittel weist dann einen Schmelzpunkt von wenigstens 40 °C, vorzugsweise wenigstens 50 °C, insbesondere wenigstens 55 °C auf und ist ein für Nahrungsmittel geeignetes Fett, das vorzugsweise unter Fettsäuren, Fettsäureestern, Fettalkoholen und Wachsen und deren Gemischen ausgewählt ist.

Beispiele für erfindungsgemäß geeignete Fette mit einem Schmelzpunkt von wenigstens 40 °C sind:
- gesättigte Fettsäuren mit 12 bis 30 C-Atomen (gesättigte C₁₂-C₃₀-Fettsäuren), insbesondere 14 bis 28 C-Atomen (gesättigte C₁₄-C₂₈-Fettsäuren) und speziell 16 bis 24 C-Atomen (gesättigte C₁₆-C₂₄-Fettsäuren) wie Myristinsäure, Palmitinsäure, Margarinsäure, Stearinsäure, Arachinsäure, Behensäure, Cerotinsäure, Melissinsäure und Lignocerinsäure sowie deren Gemische;
- Fettsäureester von gesättigten Fettsäuren mit 14 bis 30 C-Atomen, insbesondere 14 bis 28 C-Atomen und speziell 16 bis 24 C-Atomen wie die Ester der Palmitinsäure, Margarinsäure, Stearinsäure, Arachinsäure, Behensäure, Cerotinsäure, Melissinsäure und Lignocerinsäure sowie deren Gemische, insbesondere die Mono-, Di- und Triglyceride der vorgenannten Fettsäuren und deren Gemische (im Folgenden als Fettsäure-Glyceride bezeichnet), insbesondere der vorgenannten gesättigten Fettsäuren und speziell solcher gesättigter Fettsäuren mit 14 bis 28 C-Atomen und speziell 16 bis 28 C-Atomen, sowie Ester der vorgenannten gesättigten Fettsäuren mit vorzugsweise 14 bis 28 C-Atomen und speziell 16 bis 28 C-Atomen, mit C₁₀-C₃₀-Fettalkoholen, insbesondere mit C₁₄-C₂₈-Fettalkoholen. Die vorgenannten Ester von gesättigten Fettsäuren können bis zu 10 Gew.-%, bezogen auf die Fettsäureanteil im Ester, auch ein oder mehrfach ungesättigte Fettsäuren in veresterter Form enthalten. Insbesondere macht der Anteil ungesättigter Fettsäurebestandteile in diesen Estern weniger als 5 Gew.-% aus, bezogen auf die gesamten Fettsäurebestandteile im Ester;
- C₁₆-C₃₀-Fettalkohole, insbesondere gesättigte C₁₆-C₃₀-Fettalkohole, wie beispielsweise Cetylalkohol, Stearylalkohol, Nonadecanol, Arachidylalkohol, Behenylalkohol, Lignocerylalkohol, Cerylalkohol, Myricylalkohol und Melissylalkohol; sowie
- Wachse, insbesondere soweit nicht bereits in den vorgenannten Gruppen enthalten, wie Bienenwachs, Candelilawachs, Schellack-Wachs, Karitéfett und Camaubawachs, Kohlenwasserstoffwachse, wie Paraffinwachse, Ceresin, Sasolwachse, Ozokerit und Mikrowachse.

Unter den vorgenannten Fetten sind die vorgenannten Fettsäureester, insbesondere die Glyceride bevorzugt. Hierunter besonders bevorzugt sind hydrierte Pflanzenöle, tierische Öle und Fette sowie hydrierte tierische Öle und Fette.

Die feste Formulierung kann grundsätzlich beliebige Formen aufweisen. Für die erfindungsgemäße Anwendung hat es sich als vorteilhaft erwiesen, wenn die die feste Formulierung eine schüttfähige, partikelförmige Formulierung ist. Hierunter versteht der Fachmann Formulierungen, die aus einer Vielzahl diskreter Partikel bestehen. In derartigen Formulierungen weisen die Partikel typischerweise Maximalabmessungen von in der Regel nicht mehr als 1 cm auf. Vorzugsweise liegen der Mittelwert (Gewichtsmittel) der Maximalabmessungen der Partikel im Bereich von 100 bis 10000 µm, insbesondere im Bereich von 200 bis 5000 µm. Vorzugsweise weisen wenigstens 90 Gew.-% der Partikel maximale Abmessungen im Bereich von 200 µm bis 10000 µm auf. Unter einer Maximalabmessung versteht man die jeweils größte Abmessung, d.h. den Abstand der jeweils am weitest voneinander entfernten Punkte auf der Oberfläche eines Partikels. Die Form der Partikel ist dabei von untergeordneter Bedeutung. Es kann sich dabei um Schuppen, kugelförmige Partikel, zylindrische Partikel, Pastillen oder unregelmäßig geformte Partikel handeln.

Gemäß einer bevorzugten Ausführungsform der Erfindung handelt es sich bei den festen Formulierungen um Granulate. Vorzugsweise sind die Partikel des Granulats regelmäßig geformt. Häufig weisen sie eine kugelförmige, pastillenförmige oder zylindrische Form auf. Vorzugsweise liegt der Gewichtsmittlere Partikeldurchmesser der Partikel des Granulats im Bereich von 100 bis 5000 µm, insbesondere im Bereich von 200 bis 2000 µm. Vorzugsweise weisen wenigstens 90 Gew.-% der Partikel Partikeldurchmesser im Bereich von 200 bis 10000 µm auf.

Die partikelförmigen Formulierungen können ein Trenn- oder Rieselhilfsmittel enthalten, um ein Verkleben der Partikel zu vermeiden oder zu verringern. Bei den Rieselhilfsmitteln handelt es sich typischerweise um inerte, für Nahrungsmittel geeignete feste anorganische Materialien in Pulverform, z. B. um Oxide wie Aluminiumoxid, Siliciumdioxid, insbesondere in Form von feinteiliger Kieselsäure wie pyrogene Kieselsäure oder Kieselgel (E 551), oder Titandioxid (E 171), Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), oder Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (554), Kaliumaluminiumsilikat (E 555), Calciumaluminiumsilikat (E 556), Bentonit (E 558), Kaolin (E 559), Tricalciumphosphat, Natriumchlorid, und/oder um inerte, für Nahrungsmittel geeignete feste organische Materialien in Pulverform, wie beispielsweise Mono-, Di- und höhere Polysacharide wie Lactose, modifizierte Lactose, Zucker (Saccharose), Cellulose, Methylcellulose, Stearinsäure oder Stearate wie Magnesiumstearat (E 572), Ammoniumstearat (E 571) und Aluminiumstearat (E 573). Vorzugsweise weisen die Mahl oder Rieselhilfsmittel volumenmittlere Teilchengrößen im Bereich von 0,5 bis 200 µm auf. Vorzugsweise ist der mittlere Partikeldurchmesser des Rieselhilfsmittels kleiner als der mittlere Durchmesser der Partikel in der partikelförmigen Formulierung, z. B. um den Faktur 2 bis 50, insbesondere um den Faktor 4 bis 20. Der Anteil der Rieselhilfsmittel wird 10 Gew.%, bezogen auf das Gesamtgewicht der Formulierung, nicht überschreiten und beträgt, sofern vorhanden, typischerweise 0,1 bis 10 Gew.-%, vorzugsweise 0,2 bis 50 Gew.-%, insbesondere 0,3 bis 3 Gew.-%, bezogen auf das Gesamtgewicht der Formulierung.

Die festen Formulierungen können auch Fließhilfsmittel enthalten. Hierbei handelt es sich um Materialien, die ein Verkleben/Verbacken der Partikel der festen Formulierung untereinander oder mit den Behälterwänden aufgrund von Fließ- oder Schmelzprozessen an den Oberflächen der Partikel der festen Formulierung verringern. Im Unterschied zu den Trenn- oder Rieselhilfsmitteln befinden sich die Fließhilfsmittel in der festen Formulierung in suspendierter Form. Bevorzugte Fließhilfsmittel sind feinteilige Kieselsäuren, insbesondere solche mit Primärpartikelgrößen im unterhalb 500 nm, wie beispielsweise sprühgetrocknete Fällungskieselsäure, z. B. die unter der Handelsmarke Sipernat® (Evonik) vertriebenen Produkte, z. B. Sipernat® 22 S, Sipernat® 50 S, Sipemat® 500 LS, pyrogene Kieselsäuren wie die unter der Handelsmarke Aerosil® (Evonik) vertriebenen Produkte, z. B. Aerosil® 200 und Aerosil® R972 sowie amorphe Kieselgele, z. B. die unter der Handelsmarke Sylox® (Grace Davison) vertriebenen Produkte wie Sylox® 2, Sylox® 15 und Sylox® T450. Die Konzentration derartiger Fließhilfsmittel liegt typischerweise im Bereich von 0,1 und 25 Gew. -%, insbesondere im Bereich von 1,0 und 10,0 Gew.-% und besonders bevorzugt im Bereich von 1,5 und 5,0 Gew.-%, bezogen auf das Gesamtgewicht der erfindungsgemäßen Formulierung.

Alternativ kann die feste Formulierung auch grobteilig sein, beispielsweise in Form von Platten oder Blöcken. Gegebenenfalls kann man derartige Formulierungen vor der erfindungsgemäßen Anwendung zerkleinem, um ein Lösen der Formulierungen in dem flüssigen Öl zu beschleunigen.

Gemäß einer anderen Ausführungsform der Erfindung handelt es sich bei den erfindungsgemäßen Formulierungen um flüssige oder halbfeste Lösungen des Astaxanthin-Derivats. In dieser Ausführungsform ist das Lösungsmittel ein Öl und weist typischerweise einen Schmelzpunkt von höchstens 40 °C, vorzugsweise höchstens 35 °C, insbesondere höchstens 30 °C auf.

In dieser Ausführungsform ist das Lösungsmittel vorzugsweise unter pflanzlichen und tierischen Ölen, die bei Normaldruck einen Schmelzpunkt unterhalb 40 °C, vorzugsweise höchstens 35 °C und insbesondere höchstens 30 °C, z. B. im Bereich von -20 bis 40 °C, häufig -15 bis 35 °C oder -10 bis 30 °C aufweisen, ausgewählt.

Beispiele für derartige Öle sind
- flüssige Triglyceride, z. B. Pflanzenöle wie Sojabohnen-Öl, Palmöl, Palmkernöl, Sonnenblumenöl, Maiskeimöl, Leinsamenöl, Rapsöl, Distelöl, Weizenkeimöl, Reisöl, Kokosöl, Mandelöl, Aprikosenkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), tierische Öle wie Fischöle einschließlich Makrelen-, Sprotten-, Thunfisch-, Heilbutt-, Kabeljau- und Lachsöl, sowie Talgöl und semisynthetische Caprylsäure/Caprinsäure-Tricyceride wie die Miglyol-Typen; und
- für Nahrungsmittel geeignete Kohlenwasserstofföle wie Squalane, Squalene und hydrierte flüssige Polyisobutene.

Bevorzugt sind die vorgenannten Triglyceride.

Der Anteil an Lösungsmittel am Gesamtgewicht der Formulierung liegt typischerweise im Bereich von 80 bis < 98 Gew.-%, vorzugsweise im Bereich von 90 bis 97,95 Gew.-% und insbesondere im Bereich von 95 bis 97,95 Gew.-% oder 96 bis 97,95 Gew.-%, bezogen auf das Gesamtgewicht der Formulierung.

Neben dem Astaxanthin-Derivat können die erfindungsgemäßen Formulierungen auch weitere Bestandteile enthalten, wie sie für diese Zwecke üblicherweise geeignet sind und angewendet werden. Hierzu zählen lipophile Dispergiermittel, Antioxidantien (Oxidationsstabilisatoren) und dergleichen erfolgen. Beispiele für Antioxidantien sind Tocopherole wie α-Tocopherol, α-Tocopherolpalmitat, α-Tocopherolacetat, tert.-Butylhydroxytoluol, tert.-Butylhydroxytoluol, tert.-Butylhydroxyanisol, Ascorbinsäure, deren Salze und Ester wie z. B. Natriumascorbat, Calciumascorbat, Ascorbylphosphatester und Ascorbylpalmitat und Ethoxyquin. Die Antioxidantien sind, sofern erwünscht, in der erfindungsgemäßen Formulierung typischerweise in Mengen von 0,01 bis 10 Gew.-%, bezogen auf das Gesamtgewicht der Formulierung, enthalten. Typische lipophile Dispergiermittel sind Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester wie PEG(20)-Sorbitolmonooleat, Monoester der Milchsäure mit gesättigten C₁₀-C₂₄-Fettsäuren, Zuckerester von gesättigten C₁₆-C₂₈-Fettsäuren, Propylenglycol-Fettsäureester sowie Phospholipide wie Lecithin. Lipophile Dispergiermittel sind, sofern erwünscht, in der erfindungsgemäßen Formulierung typischerweise in Mengen von 0,01 bis 10 Gew.-%, bezogen auf das Gesamtgewicht der Formulierung, enthalten. Lipophile Dispergiermittel werden, sofern erwünscht, üblicherweise in Mengen von 0,1 bis 10 Gew.-Teilen, bezogen auf 1 Gew.-Teil des Astaxanthin-Derivats, eingesetzt. In einer bevorzugten Ausführungsform der Erfindung enthält die Formulierung im Wesentlichen kein, d. h. weniger als 0,5 Gew.-%, bezogen auf die Formulierung, lipophiles Dispergiermittel.

Die Herstellung der erfindungsgemäßen Formulierungen umfasst typischerweise die folgenden Schritte:
i) Bereitstellung einer Suspension eines Astaxanthin-Derivats, das zu wenigstens 80 Gew.-%, insbesondere wenigstens 90 Gew.-% und speziell zu wenigstens 96 Gew.-% aus einer Verbindung der Formel I besteht, in einem für Nahrungsmittel geeigneten organischen Lösungsmittel, das unter für Nahrungsmittel geeigneten Ölen und Fetten ausgewählt ist;
ii) Erhitzen der Suspension auf eine Temperatur von wenigstens 150 °C, z. B. auf Temperaturen im Bereich von 150 bis 220 °C, insbesondere 160 bis 210 °C und speziell im Bereich von 170 bis 200 °C; und
iii) Abschrecken der dabei erhaltenen Lösung.

Beim Erhitzen der Suspension auf die genannten Temperaturen erfolgt ein Lösen des Astaxanthin-Derivats in dem für Nahrungsmittel geeigneten Lösungsmittel bei gleichzeitiger cis-/trans-Isomerisierung auf die oben genannten Werte. Dementsprechend wird man in der Regel zur Bereitstellung der Suspension ein Astaxanthin-Derivat einsetzen, worin der Anteil des in Formel I-trans gezeigten all-trans-Isomers, bezogen auf die Gesamtmenge der in dem eingesetzten Astaxanthin-Derivat enthaltenen Verbindung der Formel I wenigstens 80 Gew.-%, insbesondere wenigstens 90 Gew.-% beträgt (z. B. 80 bis 100 Gew.-% oder 90 bis 99 Gew.-%).

In Schritt i) des erfindungsgemäßen Verfahrens wird eine Suspension des Astaxanthin-Derivats in dem für Nahrungsmittel geeigneten Lösungsmittel bereitgestellt. Vorzugsweise weisen die Partikel des Astaxanthin-Derivats in der Suspension einen volumenmittleren Teilchendurchmesser im Bereich von 0,5 bis 50 µm, insbesondere im Bereich von 0,7 bis 30 µm und speziell im Bereich von 1 bis 20 µm auf.

Unter dem volumenmittleren Teilchendurchmesser versteht man den mittels Fraunhofer-Beugung an einer verdünnten 0,01 bis 0,1 Gew.-%igen, speziell 0,05 Gew.-%igen Suspension der Kristalle in dem essbaren Öl bestimmten volumenmittleren Teilchendurchmesser (D_{4,3}-Wert).

Die Bereitstellung der Suspension des Astaxanthin-Derivats in dem für Nahrungsmittel geeignete Lösungsmittel kann in Analogie zu bekannten Verfahren zur Herstellung öliger Suspensionen von ß-Carotinoiden erfolgen, wie sie beispielsweise in dem eingangs zitierten Stand der Technik beschrieben werden. Hierzu kommen grundsätzlich Verfahren in Betracht, bei denen ein durch Fällung oder in sonstiger Weise hergestelltes Pulver des Astaxanthin-Derivats in dem für Nahrungsmittel geeigneten Öl suspendiert wird, sowie vorzugsweise Verfahren, bei denen ein festes Astaxanthin-Derivat durch Vermahlen in dem Lösungsmittel auf die gewünschte Teilchengröße gebracht wird.

Das zur Herstellung der Suspension eingesetzte, für Nahrungsmittel geeignete Lösungsmittel ist üblicherweise unter den vorgenannten Lösungsmitteln, insbesondere unter den als bevorzugt oder besonders bevorzugt genannten Lösungsmitteln ausgewählt. Das eingesetzte Lösungsmittel kann geringe Mengen an Antioxidantien und/oder lipophiler Dispergiermittel enthalten. Daneben kann das Lösungsmittel auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des eingesetzten Lösungsmittels, enthalten.

Gemäß einer bevorzugten Ausführungsform erfolgt die Bereitstellung der Suspension in Schritt i) des erfindungsgemäßen Verfahrens durch Vermahlen des Astaxanthin-Derivats, welches die oben genannte Reinheit aufweist, in einem für Nahrungsmittel geeigneten Lösungsmittel. Hierzu wird man in der Regel das Astaxanthin-Derivat zunächst in dem Lösungsmittel suspendieren, wobei man eine grobteilige Suspension des Astaxanthin-Derivats erhält, und anschließend die Partikel in einer geeigneten Vorrichtung zum Vermahlen auf die gewünschte Teilchengröße zerkleinern. Als Vorrichtungen zum Vermahlen können übliche, dem Fachmann bekannte Vorrichtungen, beispielsweise Kugelmühlen, Perlmühlen oder Kolloid-Mühlen eingesetzt werden. Das Vermahlen erfolgt typischerweise bei Temperaturen unterhalb 100 °C, häufig im Bereich von 20 bis 90 °C und insbesondere im Bereich von 30 bis 70 °C. Das Vermahlen wird so lange durchgeführt, bis die gewünschte Teilchengröße erreicht ist. Die Konzentration an Astaxanthin-Derivat in der Suspension wird in der Regel so gewählt, dass sie im Bereich von 2,5 bis 50 Gew.-%, insbesondere im Bereich von 5 bis 40 Gew.-% liegt. Dementsprechend wird man zum Vermahlen entsprechende Mengenverhältnisse an Lösungsmittel und Astaxanthin-Derivat einsetzen.

Die so erhaltene Suspension des Astaxanthin-Derivats wird in Schritt ii) des erfindungsgemäßen Verfahrens, gegebenenfalls unter Zufuhr von weiterem Lösungsmittel so lange auf eine Temperatur von wenigstens 100 °C, häufig wenigstens 120 °C, insbesondere wenigstens 150 °C, z. B. auf Temperaturen im Bereich von 100 bis 220 °C, insbesondere im Bereich von 120 bis 210 °C und speziell im Bereich von 150 bis 200 °C erhitzt, bis das Astaxanthin-Derivat weitgehend oder vollständig gelöst vorliegt und der gewünschte Isomerisierungsgrad eingestellt ist. In der Regel liegen in den Lösungen wenigstens 90 Gew.-%, insbesondere wenigstens 95 Gew.-% oder wenigstens 98 Gew.-% des in der Formulierung enthaltenen Astaxanthin-Derivats in gelöster Form vor. Die zum Lösen und Isomerisieren erforderlichen Zeiten kann der Fachmann durch geeignete Vorversuche ermitteln.

Vorzugsweise erfolgt das Lösen des Astaxanthin-Derivats möglichst rasch, so dass die mittlere Verweilzeit der dabei erhaltenen heißen Lösung in der zum Erhitzen verwendeten Vorrichtung bis zum Abkühlen/Abschrecken möglichst kurz ist und eine Dauer von 60 Sekunden, insbesondere 30 Sekunden, bevorzugt 20 Sekunden nicht überschreitet. Typischerweise wird die mittlere Verweilzeit der heißen Lösung bis zum Abkühlen/Abschrecken 0,1 bis 60 Sekunden, insbesondere 0,2 bis 30 Sekunden und speziell 0,5 bis 20 Sekunden betragen.

Im Anschluss daran wird die heiße Lösung des Astaxanthin-Derivats abgeschreckt, um Abbaureaktionen und insbesondere ein weiteres Absinken des Anteils an all-trans-Isomer zu vermeiden. Vorzugsweise wird die Temperatur nach dem Abschrecken einen Wert von 100 °C, insbesondere 80 °C und speziell 70 °C nicht überschreiten und liegt vorzugsweise im Bereich von 10 bis 100 °C, insbesondre im Bereich von 15 bis 80 °C und speziell im Bereich von 20 bis 70 °C.

Das Erhitzen kann in üblicher Weise diskontinuierlich oder kontinuierlich erfolgen. Vorzugsweise erfolgt das Erhitzen kontinuierlich, d. h. man wird einen Strom der Suspension kontinuierlich auf die gewünschte Temperatur erhitzen und nach einer Verweilzeit, die zum Lösen und gegebenenfalls Isomerisieren notwendig ist, kontinuierlich abschrecken. Das kontinuierliche Erhitzen kann beispielsweise durch Durchleiten durch Wärmetauscher, durch Einleiten von Heißdampf oder vorzugsweise durch Vermischen mit einem heißen Strom eines der vorgenannten für Nahrungsmittel geeigneten Öls erfolgen.

Gegebenenfalls ist es von Vorteil, die Suspension vor dem eigentlichen Erhitzen auf eine Temperatur vorzuwärmen, bei der ein Lösen des Astaxanthin-Derivats noch nicht stattfindet, z. B. auf Temperaturen bis 80 °C, insbesondere auf Temperaturen im Bereich von 40 bis 80 °C. Die Maximaltemperatur, bei der die Lösungsgeschwindigkeit der suspendierten Astaxanthins in der Lösungsmittelphase ausreichend langsam ist, so dass ein Lösen nicht stattfindet, hängt naturgemäß von der Größe der suspendierten Partikel sowie vom verwendeten Öl ab und kann vom Fachmann durch Routineexperimente ermittelt werden.

In einer bevorzugten Ausgestaltung erfolgt das Erhitzen durch Vermischen der Suspension, die das Astaxanthin-Derivat in einer Teilmenge des benötigten Lösungsmittels enthält, mit weiterem Lösungsmittel, das eine höhere Temperatur aufweist als die Suspension, wobei der Temperaturunterschied so gewählt ist, dass in der Mischung die zum Lösen erforderliche Temperatur resultiert. In der Regel liegt die Temperatur in der so erhaltenen Mischung im Bereich von 100 bis 220 °C, vorzugsweise im Bereich von 120 bis 210 °C und insbesondere im Bereich von 150 bis 200 °C. Die Temperaturdifferenz zwischen der Suspension und dem zugeführten Lösungsmittel beträgt in der Regel wenigstens 20 K und insbesondere wenigstens 40 K, z. B. 20 bis 220 K und insbesondere 40 bis 200 K oberhalb derjenigen Temperatur, welche die Suspension vor dem Vermischen mit der Schmelze des Verdünnungsmittels aufweist. Das Volumenverhältnis von zugeführtem Lösungsmittel und Suspension richtet sich nach der gewünschten Temperatur, der Konzentration an Astaxanthin-Derivat in der Suspension und der gewünschten Konzentration in der Lösung und liegt typischerweise im Bereich von 1000 : 1 bis 1 : 2 und speziell im Bereich von 500 : 1 bis 1 : 1.

Das Vermischen der gegebenenfalls vorgewärmten Suspension mit dem heißen Lösungsmittel kann in üblicher Weise erfolgen. Vorzugsweise wird man einen Strom der Suspension mit einem Strom des heißen Lösungsmittels in einer kontinuierlich arbeitenden Mischvorrichtung, die beispielsweise ein statischer Mischer oder eine Pumpe sein kann, vereinigen und den so erhaltenen heißen Strom, gegebenenfalls nach Durchlaufen einer vorzugsweise beheizten oder wärmeisolierten Haltestrecke zum Lösen des Astaxanthin-Derivats, abschrecken.

Bei den eingesetzten Mischvorrichtungen kann es sich um statische oder dynamische Mischer handeln, die gegebenenfalls beheizt werden können und/oder zur Vermeidung von Wärmeverlusten wärmeisoliert sind. Gegebenenfalls können sich dieser Mischzone noch eine weitere Heizzone, die mittels eines Wärmetauschers beheizt wird, und/oder eine Verweilzeit-Zone anschließen, in der das Lösen des suspendierten Astaxanthin-Derivats vervollständigt wird. Dementsprechend umfasst in dieser Ausführungsform die Heizzone im Wesentlichen eine Mischzone, in der die Suspension und das heiße Lösungsmittel miteinander vermischt werden, sowie gegebenenfalls eine weitere Heizzone, die mit einem Wärmeaustauscher oder in sonstiger Weise beheizt wird und/oder Verweilzeitzone (Haltezone), die vorzugsweise wärmeisoliert ist. Vorzugsweise umfasst die Heizzone in dieser Ausführungsform ausschließlich die Mischzone und gegebenenfalls die Verweilzeitzone.

In einer bevorzugten Ausführungsform der Erfindung erfolgt das Vermischen des heißen Lösungsmittels mit der gegebenenfalls vorgewärmten Suspension unter Verwendung einer Mischpumpe. Als Mischpumpen sind grundsätzlich alle für die Förderung von Flüssigkeit geeigneten Pumpen zu verstehen, die saugseitig zwei Anschlüsse für die zu mischenden Flüssigkeiten aufweisen. Beispiele für geeignete Mischpumpen sind insbesondere Pumpen mit rotierenden Fördermitteln wie Kreiselradpumpen, insbesondere als Peripheralpumpe bzw. Peripheralradpumpen ausgestaltete Kreiselpumpen, weiterhin Rotationspumpen wie Zahnrad- und Kreiskolbenpumpen, sowie Rotor-Stator-Mischer. In einer bevorzugten Ausführungsform der Erfindung erfolgt das Vermischen des heißen Ölstroms mit der Suspension unter Verwendung einer Peripheralpumpe, die auch als Reaktionsmischpumpe bezeichnet wird und beispielsweise unter der Bezeichnung "Reaktionsmischpumpe" kommerziell erhältlich sind, z. B. von der Fa. K-Engineering Mischtechnik und Maschinenbau, Westoverledingen DE.

Es versteht sich von selbst, dass hierbei das zur Herstellung der Suspension verwendete Lösungsmittel nicht notwendigerweise mit dem zugeführten Lösungsmittel identisch ist aber identisch sein kann. Für die Herstellung fester Lösungen hat es sich bewährt, die Suspension zunächst in einem niedrig schmelzenden Öl (Schmelzpunkt < 40 °C, insbesondere maximal 35 °C oder 30 °C, z. B. -20 bis < 40 °C, häufig -15 bis 35 °C oder -10 bis 30 °C) herzustellen und anschließend mit einer Schmelze eines höher schmelzenden Lösungsmittels (Schmelzpunkt oberhalb 40 °C, insbesondere im Bereich von 40 bis 100 °C) zu vermischen.

Das Abschrecken der noch heißen Lösung des Astaxanthin-Derivats kann grundsätzlich in üblicher Weise erfolgen.

Bei der Herstellung fester Formulierungen (d. h. das Lösungsmittel weist einen Schmelzpunkt von wenigstens 40 °C auf) kann man die noch flüssige Lösung durch Ausgießen auf gekühlte Walzen oder Platten abschrecken, wobei sich eine feste Schicht bildet, die anschließend zerkleinert werden kann. Alternativ kann man auch ein so genanntes Prilling, Jet-Prilling oder Kryoprilling der flüssigen Lösung durchführen. Hierzu wird man die geschmolzene Lösung in Tröpfchen überführen, die man mittels eines kühlen Gasstroms, beispielsweise mittels eines kühlen Stickstoffstroms, oder einer kühlen Flüssigkeit, in der das Verdünnungsmittel unlöslich ist, beispielsweise Wasser, abschreckt. Ebenfalls ist es möglich, so genannte Pastillierverfahren anzuwenden, bei denen man typischerweise die geschmolzene Lösung auf gekühlte Flächen, z. B. gekühlte rotierende Teller oder Platten, tropft und anschließend von der gekühlten Fläche mittels geeigneter Vorrichtungen entfernt.

Im Falle von flüssigen Formulierungen (d. h. das Lösungsmittel weist einen Schmelzpunkt von maximal 40 °C oder darunter auf), wird man vorzugsweise so vorgehen, dass man die heiße Lösung mit einem kühleren, für Nahrungsmittel geeigneten Lösungsmittel vermischt.

Die Temperatur des kühleren Lösungsmittels wird in der Regel wenigstens 50 K, häufig wenigstens 80 K und insbesondere wenigstens 100 K unterhalb der Temperatur liegen, welche die heiße Lösung nach Beendigung des Erhitzens aufweist. Sofern das Abschrecken weitere Maßnahmen zur Kühlung umfasst, kann die Temperaturdifferenz auch weniger als 50 K betragen, in der Regel jedoch wenigstens 10 K, häufig wenigstens 20 K, insbesondere wenigstens 30 K.

Typischerweise wird man die Menge und Temperatur des zum Abschrecken verwendeten kühleren Lösungsmittels so wählen, dass die Temperatur der durch Vermischen erhaltenen Lösung nicht mehr als 80 °C, insbesondere nicht mehr als 70 °C und speziell nicht mehr als 60 °C beträgt und beispielsweise im Bereich von 10 bis 80 °C, insbesondere im Bereich von 15 bis 70 °C und speziell im Bereich von 20 bis 60 °C liegt. Typischerweise weist das zum Abschrecken verwendete kühlere Lösungsmittel eine Temperatur im Bereich von 10 bis 60 °C und insbesondere im Bereich von 20 bis 50 °C und speziell im Bereich von 20 bis 30 °C auf.

Das Volumenstromverhältnis bzw. das Verhältnis der Flussraten von heißer Lösung zu der kühlerem Lösungsmittel liegt typischerweise im Bereich von 1 : 500 bis 1 : 1 und speziell im Bereich von 1 : 300 bis 1 : 2.

Das Vermischen der heißen Lösung mit dem kühleren Lösungsmittel kann in beliebiger Weise erfolgen, z. B. durch Eintragen der heißen Lösung in einen großen Überschuss des kühleren Lösungsmittels oder vorzugsweise durch Einspeisen eines Volumenstroms des kühleren Lösungsmittels in den Strom der heißen Lösung.

Das Einspeisen des kühleren Lösungsmittels in die heiße Lösung kann in üblicher Weise unter Verwendung von Mischvorrichtungen, die statisch oder dynamisch ausgestaltet sein können, erfolgen. In einer bevorzugten Ausführungsform der Erfindung erfolgt das Einspeisen des kühleren Lösungsmittels in die heiße Lösung unter Verwendung einer dynamischen Mischvorrichtung, insbesondere unter Verwendung einer Mischpumpe. Für diesen Zweck geeignete Mischpumpen sind die zuvor im Zusammenhang mit dem Vermischen von Suspension und heißem Ölstrom genannten Mischpumpen, insbesondere Peripheralpumpen bzw. Reaktionsmischpumpen. Die Verwendung von Mischpumpen führt zu einer besonders raschen Abkühlung der heißen Lösung, und ein Auskristallisieren des Astaxanthin-Derivats wird vollständig vermieden.

Das kühlere Lösungsmittel kann gleich dem zur Herstellung der Suspension bzw. dem beim Erhitzen zugeführten Lösungsmittel oder davon verschieden sein. Die Art des Lösungsmittels ist für die Erfindung von untergeordneter Bedeutung, so dass es grundsätzlich aus allen der vorgenannten flüssigen bzw. niedrig schmelzenden Lösungsmittel ausgewählt sein kann. Vorzugsweise ist das kühlere Lösungsmittel unter Pflanzenölen wie Sojabohnen-Öl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, MCT-Ölen, weiterhin Fischölen, Fischölblends sowie Mischungen dieser Öle ausgewählt, insbesondere unter einem Öl oder einer Ölmischung aus der folgenden Gruppe: Maisöl, Sonnenblumenöl, Sojabohnenöl, Fischöl, Leinsamenöl, Rapsöl, Reisöl, Kokosöl und Erdnussöl.

Anstelle des kühleren Lösungsmittels kann man auch eine kühlere Lösung des Astaxanthin-Derivats in dem für Nahrungsmittel geeigneten Lösungsmittel verwenden, die aus einer vorangegangenen Herstellung stammt oder durch Rückführen eines Teilstroms der abgeschreckten Lösung stammt. Derartige Verfahren sind in der älteren PCT/EP2008/065989 und der älteren PCT/EP2008/065991 für die Herstellung von Lösungen des Astaxanthins beschrieben und können zur Herstellung der erfindungsgemäßen Lösungen angewendet werden.

Gegebenenfalls kann das Abschrecken der heißen Lösung weitere Maßnahmen zum Abkühlen umfassen. Zu nennen sind beispielsweise Wärmetauscher, welche vorzugsweise dem Vermischen der heißen Lösung des Astaxanthin-Derivats mit dem kühleren Lösungsmittel nachgeschaltet sind.

Die erfindungsgemäßen Formulierungen lassen sich einfach in flüssige, für Nahrungsmittel geeignete Öle einarbeiten und eigenen sich daher in besondere Weise zur Herstellung von Futtermittelzubereitungen, insbesondere festen Futtermittelzubereitungen, speziell Futtermittelzubereitungen in Form von Pellets.

Dementsprechend betrifft die vorliegende Erfindung auch ein Verfahren zur Herstellung von Futtermittelzubereitungen, insbesondere von festen Futtermittelzubereitungen, speziell von Pellets, umfassend die folgenden Schritte:
a) Bereitstellung einer erfindungsgemäßen Formulierung wie hier beschrieben;
b) Verdünnen der Formulierung mit einem für Nahrungsmittel geeigneten, unter Prozessbedingungen flüssigen Öl unter Erhalt einer verdünnten flüssigen Lösung des Astaxanthin-Derivats in dem flüssigen Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

Unter "Prozessbedingungen flüssig" bedeutet, dass das für Nahrungsmittel geeignete Öl unter den Bedingungen des Einarbeitens der in Schritt b) erhaltenen Lösung in die Bestandteile der Futtermittelzusammensetzung in Schritt c) flüssig ist.

Das in Schritt b) eingesetzte Öl kann synthetischer, mineralischer, pflanzlicher oder tierischer Herkunft sein. Beispiel sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl und PUFA-Öle (PUFA = mehrfach ungesättigte Fettsäuren (polyunsaturated fatty acids) wie Eikosapentaensäure (EPA), Docosahexaensäure (DHA) und α-Linolensäure), Triglyceride mittelkettiger (= C₈-C₁₀) pflanzlicher Fettsäuren (sog. MCT-Öle), weiterhin semisynthetische Triglyceride, z. B. Caprylsäure/Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl und Lanolin.

Bevorzugt sind pflanzliche Öle und Öle tierischen Ursprungs, die bei 40 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, weiterhin Fischöle, sowie Mischungen dieser Öle.

In einer bevorzugten Ausführungsform der Erfindung umfasst das in Schritt b) eingesetzte Öl zu wenigstens 50 Gew.-% und insbesondere zu wenigstens 80 Gew.-%, bezogen auf die Gesamtmenge des in Schritt b) eingesetzten essbaren Öls, wenigstens ein unter Maisöl, Sonnenblumenöl, Sojabohnenöl und Fischöl ausgewähltes essbares Öl.

Bei den in Schritt b) eingesetzten Ölen kann es sich um Raffinatöle oder Rohöle handeln, die noch ursprungsspezifische Verunreinigungen wie Proteine, Phosphat, (Erd)alkalimetallsalze und dergleichen in üblichen Mengen enthalten. Die in Schritt b) eingesetzten Öle können auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des in Schritt b) eingesetzten Öls, enthalten.

Das Vermischen des flüssigen Öls mit der erfindungsgemäßen Formulierung erfolgt in der Regel bei Temperaturen oberhalb des Schmelzpunktes des in der Formulierung enthaltenen Lösungsmittels, wobei in der Regel eine Temperatur ausreicht die wenigstens 1 K, insbesondere wenigstens 5 K oberhalb des Schmelzpunktes des in der Formulierung enthaltenen Verdünnungsmittels liegt. In der Regel wird man beim Einarbeiten der erfindungsgemäßen Formulierung in das flüssige Öl eine Temperatur unterhalb 100 °C einhalten. Das Einarbeiten der erfindungsgemäßen Formulierung in das flüssige Öl erfolgt typischerweise bei Temperaturen von maximal 100 °C, vorzugsweise bei Temperaturen von nicht mehr als 95 °C und speziell nicht mehr als 90 °C und vorzugsweise bei Temperaturen im Bereich von 10 bis 100 °C, insbesondere im Bereich von 15 bis 95 °C und speziell im Bereich von 20 bis 90 °C.

Typischerweise wird man die relativen Mengen an Öl und erfindungsgemäßer Formulierung so wählen, dass der Gehalt an Astaxanthin-Derivat in der resultierenden Lösung im Bereich von 10 ppm (= 0,001 Gew.-%) bis etwa 1 Gew.-%, häufig im Bereich von 20 ppm bis 0,5 Gew.-%, insbesondere im Bereich von 50 ppm bis 0,2 Gew.-%, bezogen auf die Gesamtmenge an Öl plus Astaxanthin-Derivat liegt.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die erfindungsgemäße Formulierung unter Durchmischen zugibt, wobei die Zugabe in einer Portion, in mehreren Portionen oder kontinuierlich erfolgen kann. Die zugegebene feste Formulierung des Astaxanthin-Derivats kann auf die gewünschte Temperatur vorgewärmt werden, was jedoch grundsätzlich nicht erforderlich ist.

Das Einarbeiten der erfindungsgemäßen Formulierung in das für Nahrungsmittel geeignete Öl kann auch kontinuierlich durch sog. Inline-Mischer, d. h. kontinuierlich arbeitende Mischvorrichtungen, z. B. statische Mischer oder dynamische Mischer, erfolgen. Beispiele für geeignete statische Mischer sind Mischkammem mit oder ohne Einbauten, Mischrohre mit oder ohne Mischdüsen, Jet-Mischer und der gleichen. Beispiele für dynamische Mischer sind Rotor-Stator-Mischer, Mischkammem mit Rührwerken, Mischpumpen und dergleichen. In diesem Fall wird man die feste Formulierung aufschmelzen. Gegebenenfalls schließt sich der Mischvorrichtung noch eine Verweilzone an, die gegebenenfalls temperiert werden kann, um das Lösen des Astaxanthin-Derivats zu vervollständigen.

Die zum Erreichen des Lösens erforderliche Mischdauer hängt naturgemäß von der Temperatur, dem gewählten Öl und den apparativen Gegebenheiten ab und liegt typischerweise im Bereich von 10 sec. bis 20 min. Der Fachmann kann die erforderliche Mischzeit durch Routineexperimente ermitteln.

Auf diese Weise erhält man stabile Lösungen von Astaxanthin-Derivaten in für Nahrungsmittel geeigneten, bei Prozessbedingungen flüssigen Ölen. Die so erhaltenen Lösungen enthalten neben dem Astaxanthin-Derivat die in der Formulierung enthaltenen Hilfsstoffe, wie ggf. Oxidationsstabilisatoren, lipophile Dispergiermittel, Fließhilfsmittel und/oder Verdicker.

Es versteht sich von selber, dass die erfindungsgemäß erhältlichen Lösungen geringe Mengen an Wasser aus dem in Schritt b) eingesetzten, für Nahrungsmittel geeigneten Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öl.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermitteln, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Lösungen des Astaxanthin-Derivats. Diese Mittel enthalten neben den für diese Mittel üblichen Bestandteilen das in Schritt b) eingesetzte Öl, das Astaxanthin-Derivat sowie die weiteren Bestandteile der erfindungsgemäßen Formulierung.

Bevorzugte Ausführungsformen betreffen Tierfuttermittel insbesondere Fischfuttermittel, die das flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxanthin-Derivat die oben genannten Eigenschaften aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthaltend das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

Die folgenden Figuren und Beispiele dienen der weiteren Erläuterung der Erfindung.

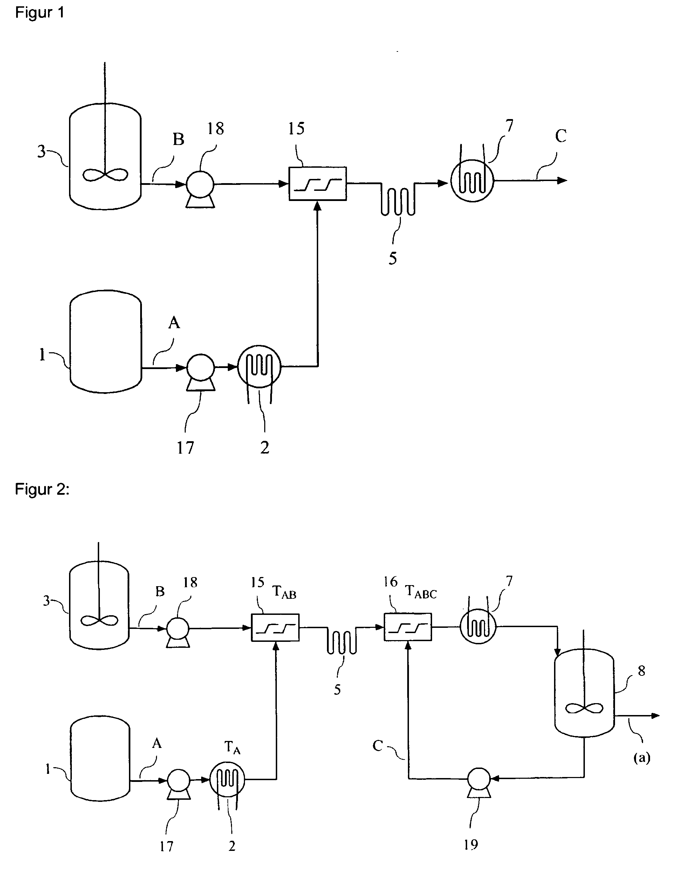

Die Bezugszeichen in den Figuren 1 und 2 haben folgende Bedeutungen:
- (1): Vorlagenbehälter für Lösungsmittel
- (2): Wärmetauscher
- (3): Vorlagenbehälter für Suspension des Astaxanthin-Derivats (z. B. Ax-DMDS)
- (5): Verweilzeitstrecke
- (7): Wärmetauscher
- (8): Vorlagenbehälter für kühlere Lösung des Astaxanthin-Derivats (z. B. Ax-DMDS)
- (9): Zuleitung für frisches Öl
- (15): Mischvorrichtung, bzw. statischer Mischer
- (16): Mischvorrichtung, bzw. statischer Mischer
- (17): Förderpumpe
- (18): Förderpumpe
- (19): Förderpumpe
- (a): Produktentnahme

- A: Strom Lösungsmittel
- B: Suspensionsstrom
- C: Strom der Lösung des Astaxanthin-Derivats (Produktstrom)

- T_{A}: Temperatur im Wärmetauscher (2)
- T_{AB}: Temperatur im statischen Mischer (15)
- T_{ABC}: Temperatur im statischen Mischer (16)

Figur 1 zeigt schematisch ein semikontinuierliches Verfahren zur Herstellung einer Lösung von Ax-DMDS in einem für Nahrungsmittel geeigneten Lösungsmittel durch Erhitzen einer Suspension von Ax-DMDS in einem für Nahrungsmittel geeigneten Lösungsmittel, bei der das Erhitzen mittels eines heißen Lösungsmittelstroms vorgenommen wird.

Gemäß dem in Figur 1 dargestellten Verfahren wird ein für Nahrungsmittel geeignetes Lösungsmittel aus dem Vorlagenbehälter (1) mittels einer Förderpumpe (17) in den Wärmetauscher (2) geleitet und dort auf die zum Erhitzen erforderliche Temperatur T_{A} erhitzt, insbesondere auf Temperaturen im Bereich von 160 bis 210 °C. Parallel hierzu wird aus dem Vorlagenbehälter (3), welcher Mittel zum Bewegen der Suspension wie Rührer, etc. aufweist, ein Strom B einer Ax-DMDS-Suspension, die eine Temperatur T_{B} aufweist, mittels einer Förderpumpe (18) in den statischen Mischer (15) geleitet und dort mit dem auf die Temperatur T_{A} erhitzten Strom zusammengeführt. Hierbei stellt sich in der Mischung (= Strom AB), abhängig vom Verhältnis der Volumenströme A und B die Mischtemperatur T_{AB} ein, die typischerweise im Bereich von 120 bis 200 °C, insbesondere im Bereich von 150 bis 190 °C liegt. Konzentration im Strom AB liegt vorzugsweise im Bereich von 0,2 bis 10 g/kg. Das Volumenstromverhältnis von Strom A zu Strom B wird vorzugsweise im Bereich von 100 : 1 bis 1 : 1 und insbesondere im Bereich von 50 : 1 bis 2 : 1 liegen. Die auf die Temperatur T_{AB} erhitzte Mischung AB passiert die wärmeisolierter Haltestrecke (5) und wird von dort im Wärmetauscher (7) auf eine Temperatur im Bereich von 20 bis 70 °C abgekühlt und als Produktstrom C ausgeschleust.

Figur 2 zeigt schematisch ein semikontinuierliches Verfahren zur Herstellung einer Lösung von Ax-DMDS in einem für Nahrungsmittel geeigneten Öl durch Erhitzen einer Suspension von Ax-DMDS in einem für Nahrungsmittel geeigneten Öl, bei der das Erhitzen analog Figur 1 mittels eines heißen Lösungsmittelstroms vorgenommen wird und bei dem zum Quenchen ein Lösungsmittel verwendet wird, bei dem es sich um eine kühlere Lösung des Ax-DMDS in einem für Nahrungsmittel geeigneten Lösungsmittel handelt, die durch Rückführen der beim Quenchen erhaltenen Lösung in ein Vorratsgefäß, das mit einem für Nahrungsmittel geeigneten Öl befüllt ist, hergestellt wird.

Im Unterschied zu dem in Figur 1 gezeigten Verfahren wird die auf die Temperatur T_{AB} erhitzte Mischung AB nach passieren der wärmeisolierter Haltestrecke (5) in den statischen Mischer (16) geleitet und mit einem kalten Strom C einer Lösung des Ax-DMDS in dem essbaren Öl, die eine Temperatur Tc aufweist, zum Strom ABC vereinigt. Hierbei stellt sich, abhängig vom Verhältnis der Volumenströme C und AB schlagartig die Mischtemperatur T_{ABC} ein, die typischerweise im Bereich von 30 bis 90 °C, insbesondere im Bereich von 35 bis 80 °C liegt. Der Strom ABC wird anschließend über einen Wärmeüberträger (7) auf Umgebungstemperatur abgekühlt und in eine Sammelgefäß (8) gegeben, das Mittel zum Durchmischen von Flüssigkeiten wie Rührer, etc. aufweist und in dem zu Beginn Reaktion ein essbares Öl oder eine Lösung des Ax-DMDS vorgelegt wurde. Aus dem Vorratsgefäß (8) wird mittels einer Pumpe (19) der Strom C entnommen und dem Mischer (16) zugeführt. Mit zunehmender Versuchsdauer nähert sich die Konzentration des Ax-DMDS in der im Sammelbehälter befindlichen Lösung der durch diese Mischung von Strom A und Strom B vorgegebenen maximalen Mischkonzentration an.

### Analytik:

Die Bestimmung der Partikelgrößen erfolgte mittels Fraunhoferbeugung unter Verwendung eines Malvern Mastersizer S bei 22 °C. Die durch Vermahlen erhaltenen Suspensionen des Astaxanthin-Derivats wurden vor der Messung mit dem zum Vermahlen verwendeten Öl auf eine Konzentration von 0,05 Gew.-% verdünnt. Angegeben ist der volumenmittlere Teilchendurchmesser D_{4,3}.

Zur Bestimmung des absoluten Gehalts an Astaxanthin-Derivat in den zur Herstellung der Lösungen verwendeten Suspensionen löste man die Suspension in Aceton und bestimmte anschließend mittels UV/VIS-Spektroskopie (200 - 800 nm) die Extinktion der Lösung bei 478 nm.

Zur Bestimmung des Gehalts an gelöstem Astaxanthin in den erfindungsgemäß erhältlichen Lösungen, filtrierte man die Proben über einen Filter mit einer Porenweite von 0,2 µm und bestimmte anschließend mittels UV/VIS-Spektroskopie (200 - 800 nm) die Extinktion der Lösung bei 478 nm.

Die Bestimmung des Anteils an all-trans-Isomer erfolgte mittels HPLC.

In den folgenden Versuchen wurde das in Beispiel 5 der WO 03/066583 beschriebene (all-E)-3,3'-rac-Astaxanthindimethyldisuccinat (Ax-DMDS) in kristalliner Form mit einer Reinheit > 96 % eingesetzt
(Verbindung der Formel I, worin R für C(=O)CH₂CH₂C(=O)OCH₃ steht).

Zur Herstellung der Suspensionen Lösungen wurden die folgenden handelsüblichen Öle eingesetzt:
Sonnenblumenöl: handelsübliches Produkt mit Lebensmittelqualität der Fa. Gustav Heess GmbH; Wassergehalt < 0,1 %; Fettsäureverteilung: 4-9 % Palmitinsäure, 1-7 % Stearinsäure, 14-40 % Ölsäure, 48-70 % Linolsäure.
Palmkernöl: handelsübliches Raffinat mit Lebensmittelqualität; Wassergehalt < 0,1 %; Gehalt an C12/C14-Carbonsäuretriglycieriden > 60 %.
MCT-Öl: Delios® MCT-Öl der Fa. Cognis GmbH; Wassergehalt < 0,1 %, Gehalt an C8/C10-Fettsäuretriglyceriden > 95 %.
PUFA-Öl: handelsübliches Produkt mit Lebensmittelqualität der Fa. Napro-Pharma AS, Norwegen; Eicosahexaensäure > 250 mg/g, Eicosapentaesäure < 57 mg/g, bestimmt nach European Pharmacoepia 2.4.29.

### Allgemeine Vorschrift für das Vermahlen von Ax-DMDS

300 g Ax-DMDS wurden mit 2700 g eines für Nahrungsmittel geeigneten Öls (MCT-Öl) vermischt, so dass man eine 10 gew.-%ige Primärsuspension erhielt. Diese Primärsuspension wurde anschließend bei Temperaturen im Bereich von 20 bis 25 °C mit einem Druck von 0,5 bar, einer Durchflussrate von 60 bis 70 g/min in einer Kugelmühle (Drais PML1 A/V: Sieb, 0,4 mm, 3270 U/min, Mahlraumvolumen 1000 ml, gefüllt mit ZrO₂-stablisierten Kugeln mit einem Durchmesser von 0,7 bis 1,2 mm) vermahlen. Der Teilchendurchmesser der Suspendierten Ax-DMDS-Partikel wurde nach 0,5 h und 4 h Mahldauer bestimmt. Die Ergebnisse sind in der folgenden Tabelle zusammengefasst:

| Laufzeit | X₅₀ [µm] | X₉₀ [µm] |
|---|---|---|
| 0,5 h | 1,0 | 5,1 |
| 4h | 0,071 | 0,97 |

### Beispiel 1:

1. 14,2 g Ax-DMDS wurden mit 85,8 g MCT-Öl (Delios der Fa. Cognis) vermischt so dass man eine 14,2 gew.-%ige Primärsuspension erhielt. Diese Primärsuspension wurde 6 h in einer konventionellen Schüttelmühle (RedDevil®) vermahlen. Die erhaltene Suspension wies einen mittleren Partikeldurchmesser d_{4,3} im Bereich von 1 - 10 µm auf.
2. Die in Schritt 1 erhaltene Suspension von Ax-DMDS in MCT-Öl wurde in der in Figur 1 gezeigten Anordnung in MCT-Öl gelöst. Hiezu wurde MCT-Öl aus dem beheizten Vorlagenbehälter (1) mittels einer Zahnradpumpe (17) mit einer Geschwindigkeit von 2,26 kg/h gefördert und mittels eines Ölbades (2) auf 220 °C erhitzt. Parallel hierzu wurde aus dem Vorlagenbehälter (3) ein Strom B der in Schritt 1 hergestellten Suspension, die eine Temperatur von 22 °C aufweist, mittels einer Zahnradpumpe (18) mit einer Geschwindigkeit von 0,72 kg/h gefördert. Die Ströme A und B wurden in einem ersten T-Stück als statischer Mischer (15) zusammengeführt und dabei intensiv durchmischt. Das T-Stück wies in allen Rohren einen Innendurchmesser von 0,75 mm auf. Hierbei stellte sich in der Mischung (= Strom AB) die Mischtemperatur von etwa 163,8 °C ein. Die so erhitzte Mischung passierte eine wärmeisolierter Haltestrecke (5) mit einer durchschnittlichen Verweilzeit von 12,3 sec. und wurde von dort in einem Wärmetauscher geleitet und auf eine Temperatur 20 °C abgekühlt. Die erhaltene Formulierung wies einen Gehalt an Ax-DMDS von 2,8 Gew.-% auf. Das enthaltene Astaxanthin war vollständig gelöst (< 99,5 %). Der all-trans-Anteil lag bei 63 %. Die erhaltene Lösung wies auch nach 30 Tagen keine Feststoffe auf.

## Patentansprüche

1. Formulierung von Astaxanthin-Derivaten in Form einer Lösung des Astaxanthin-Derivats in einem für Nahrungsmittel geeigneten organischen Lösungsmittel, das unter für Nahrungsmittel geeigneten Ölen, Fetten und Wachsen und deren Gemischen ausgewählt ist, mit einem Gehalt an Astaxanthin-Derivat von mehr als 2 Gew.-%, bezogen auf das Gesamtgewicht der Formulierung,
wobei das Astaxanthin-Derivat zu wenigstens 80 Gew.-% aus wenigstens einer Verbindung der Formel I 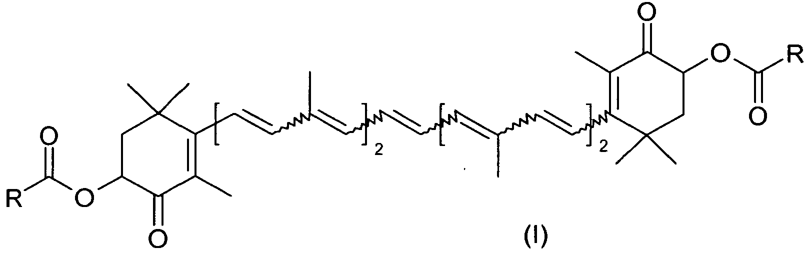 besteht, worin R für einen Rest der Formel 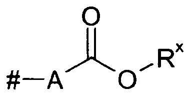 steht, worin # die Anknüpfung an die Carbonylgruppe bedeutet, A für -CH₂CH₂-oder für -CH=CH- steht und Rₓ für C₁-C₄ Alkyl steht, wobei das Anteil des all-trans-Isomeren (I-trans), bezogen auf die Gesamtmenge der in der Lösung enthaltenen Verbindung der Formel I weniger als 80 % beträgt.

2. Formulierung nach Anspruch 1, enthaltend das Astaxanthin-Derivat in einer Menge von 2,05 bis 4 Gew.-%.

3. Formulierung nach Anspruch 1 oder 2, wobei in Formel I der Rest R^{x} Methyl oder Ethyl bedeutet und A für -CH₂CH₂- steht.

4. Formulierung nach einem der vorhergehenden Ansprüche, wobei das für Nahrungsmittel geeignete organische Lösungsmittel ausgewählt ist unter Fettsäuren, Mono-, Di- und Triglyceriden von Fettsäuren, Fettalkoholen, Estern von Fettsäuren mit Fettalkoholen und deren Gemischen.

5. Formulierung nach Anspruch 4, wobei das für Nahrungsmittel geeignete organische Lösungsmittel ein Fett ist, das bei Normaldruck einen Schmelzpunkt von wenigstens 40 °C aufweist.

6. Formulierung nach Anspruch 5 in Form eines Granulats.

7. Formulierung nach Anspruch 4, wobei das für Nahrungsmittel geeignete organische Lösungsmittel ausgewählt ist unter pflanzlichen und tierischen Ölen, die bei Normaldruck einen Schmelzpunkt unterhalb 40 °C aufweisen.

8. Formulierung nach einem der vorhergehenden Ansprüche, enthaltend weiterhin wenigstens einen weiteren für Nahrungsmittel geeigneten Zusatz, der unter Oxidationsstabilisatoren, Emulgatoren, Fließhilfsmitteln und Mitteln zur Erhöhung der Viskosität ausgewählt ist.

9. Verfahren zur Herstellung einer Formulierung gemäß einem der vorhergehenden Patentansprüche, umfassend die folgenden Schritte:
i) Bereitstellung einer Suspension eines Astaxanthin-Derivats, das zu wenigstens 80 Gew.-% aus einer Verbindung der Formel I gemäß Anspruch 1 oder 2 besteht, in einem für Nahrungsmittel geeigneten organischen Lösungsmittel, das unter für Nahrungsmittel geeigneten Ölen und Fetten ausgewählt ist;
ii) Erhitzen der Suspension auf eine Temperatur von wenigstens 100 °C; und
iii) Abschrecken der in Schritt ii erhaltenen Lösung.

10. Verfahren nach Anspruch 9, wobei man die Suspension in Schritt i) durch Vermahlen des Astaxanthin-Derivats in dem für Nahrungsmittel geeigneten Öl, Fett oder Wachs bereitstellt.

11. Verfahren nach Anspruch 9 oder 10, wobei die Partikel des Astaxanthin-Derivats in der Suspension einen volumenmittleren Teilchendurchmesser, bestimmt durch Fraunhoferbeugung, im Bereich von 0,5 bis 50 µm aufweisen

12. Verfahren nach einem der Ansprüche 9 bis 11, wobei man die in Schritt ii. erhaltene Lösung mittels eines kühlen Gasstroms oder einer kühlen Flüssigkeit, in der das Verdünnungsmittel unlöslich ist, abschreckt.

13. Verfahren nach einem der Ansprüche 9 bis 11, wobei man die in Schritt ii. erhaltene Lösung mittels eines kühlen Stroms eines für Nahrungsmittel geeigneten Lösungsmittels und/oder mittels eines Wärmetauschers abschreckt.

14. Verwendung einer Formulierung nach einem der Ansprüche 1 bis 8 zur Herstellung von Futtermittelzubereitungen, insbesondere festen Futtermittelzubereitungen, speziell Futtermittelzubereitungen in Form von Pellets.

15. Verfahren zur Herstellung von Futtermittelzubereitungen, insbesondere von festen Futtermittelzubereitungen, speziell von Pellets, umfassend:
a) Bereitstellung einer Formulierung gemäß einem der Ansprüche 1 bis 8;
b) Verdünnen der Formulierung mit einem für Nahrungsmittel geeigneten flüssigen Öl unter Erhalt einer verdünnten flüssigen Lösung des Astaxanthin-Derivats in dem flüssigen Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

16. Verfahren nach Anspruch 14, wobei das Lösungsmittel ausgewählt ist unter pflanzlichen und tierischen Ölen, die bei Normaldruck einen Schmelzpunkt unterhalb 40 °C aufweisen.

17. Verfahren nach Anspruch 15 oder 16, wobei die in Schritt b) erhaltene verdünnte flüssige Lösung das Astaxanthin-Derivat in einer Konzentration von 10 bis 1000 ppm enthält.

## Claims

1. A formulation of astaxanthin derivatives in the form of a solution of the astaxanthin derivative in an organic solvent suitable for nourishments which is selected from oils, fats and waxes suitable for nourishments and mixtures thereof, having a content of astaxanthin derivative of greater than 2% by weight, based on the total weight of the formulation, wherein the astaxanthin derivative comprises at least 80% by weight of at least one compound of the formula I 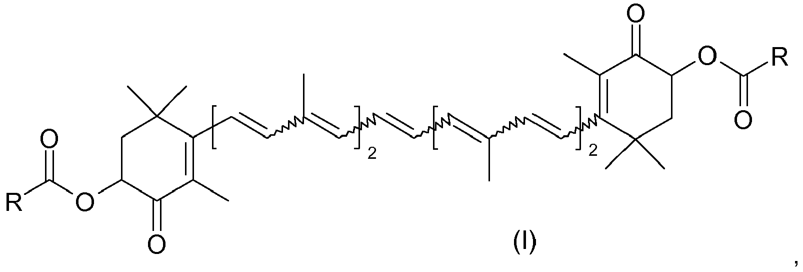 where R is a radical of the formula 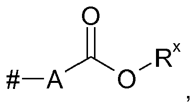 where # indicates the linkage to the carbonyl group, A is -CH₂CH₂- or - CH=CH- and R^{x} is C₁-C₄ alkyl, wherein the fraction of the all-trans isomer (I-trans), based on the total amount of the compound of the formula I present in the solution is less than 80%.

2. The formulation according to claim 1, comprising the astaxanthin derivative in an amount of 2.05 to 4% by weight.

3. The formulation according to claim 1 or 2, wherein, in formula I, the radical R^{x} is methyl or ethyl and A is -CH₂CH₂-.

4. The formulation according to any one of the preceding claims, wherein the organic solvent suitable for nourishments is selected from fatty acids, mono-, di- and triglycerides of fatty acids, fatty alcohols, esters of fatty acids with fatty alcohols, and mixtures thereof.

5. The formulation according to claim 4, wherein the organic solvent suitable for nourishments is a fat which at atmospheric pressure has a melting point of at least 40°C.

6. The formulation according to claim 5 in the form of granules.

7. The formulation according to claim 4, wherein the organic solvent suitable for nourishments is selected from vegetable and animal oils which have a melting point below 40°C at atmospheric pressure.

8. The formulation according to any one of the preceding claims, further comprising at least one further additive suitable for nourishments which is selected from oxidation stabilizers, emulsifiers, free-flowing agents and compositions for increasing the viscosity.

9. A process for producing a formulation according to any one of the preceding claims, comprising the following steps:
i) providing a suspension of an astaxanthin derivative which comprises at least 80% by weight of a compound of the formula I according to claim 1 or 2 in an organic solvent suitable for nourishments, which solvent is selected from oils and fats suitable for nourishments;
ii) heating the suspension to a temperature of at least 100°C; and
iii) quenching the solution obtained in step ii.

10. The process according to claim 9, wherein the suspension in step i) is provided by milling the astaxanthin derivative in the oil, fat or wax suitable for nourishments.

11. The process according to claim 9 or 10, wherein the particles of the astaxanthin derivative in the suspension have a volume-median particle diameter determined by Fraunhofer diffraction in the range from 0.5 to 50 µm.

12. The process according to any one of claims 9 to 11, wherein the solution obtained in step ii is quenched by means of a cold gas stream or a cold liquid in which the diluent is insoluble.

13. The process according to any one of claims 9 to 11, wherein the solution obtained in step ii is quenched by means of a cold stream of a solvent suitable for nourishments and/or by means of a heat exchanger.

14. The use of a formulation according to any one of claims 1 to 8 for producing feed preparations, in particular solid feed preparations, especially feed preparations in the form of pellets.

15. A process for producing feed preparations, in particular solid feed preparations, especially pellets, which comprises:
a) providing a formulation according to any one of claims 1 to 8;
b) diluting the formulation with a liquid oil suitable for nourishments, obtaining a dilute liquid solution of the astaxanthin derivative in the liquid oil; and
c) mixing the liquid solution obtained in step b) with the components of the feed preparation, or impregnating a solid feed preparation with the liquid solution obtained in step b).

16. The process according to claim 14, wherein the solvent is selected from vegetable and animal oils which have a melting point below 40°C at atmospheric pressure.

17. The process according to claim 15 or 16, wherein the dilute liquid solution obtained in step b) comprises the astaxanthin derivative at a concentration of 10 to 1000 ppm.

## Revendications

1. Formulation de dérivés d'astaxanthine sous forme d'une solution du dérivé d'astaxanthine dans un solvant organique approprié pour des produits alimentaires, qui est choisi parmi des huiles, graisses et cires appropriées pour des produits alimentaires et des mélanges de celles-ci, ayant une teneur en dérivé d' astaxanthine de plus de 2 % en poids, par rapport au poids total de la formulation,
le dérivé d'astaxanthine consistant à raison d'au moins 80 % en poids en au moins un composé de formule I 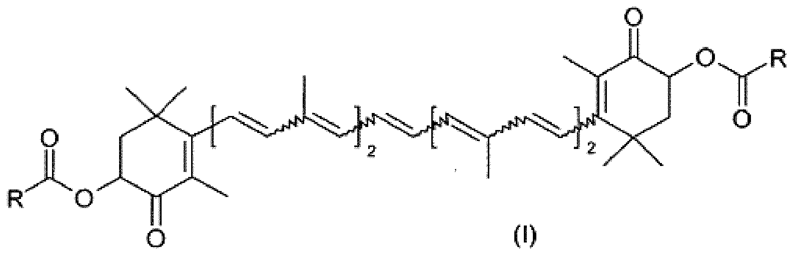 dans laquelle R représente un radical de formule 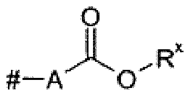 dans laquelle # représente la liaison au groupe carbonyle, A représente -CH₂CH₂- ou -CH=CH- et R^{x} représente un groupe alkyle en C₁-C₄, la proportion de l'isomère tout-trans (i-trans), par rapport à la quantité totale du composé de formule I contenu dans la solution, étant inférieure à 80 %.

2. Formulation selon la revendication 1, contenant le dérivé d'astaxanthine en une quantité de 2,05 à 4 % en poids.

3. Formulation selon la revendication 1 ou 2, dans laquelle le radical R^{x} dans la formule I représente le groupe méthyle ou éthyle et A représente -CH₂CH₂-.

4. Formulation selon l'une quelconque des revendications précédentes, dans laquelle le solvant organique approprié pour des produits alimentaires est choisi parmi des acides gras, des mono-, di- et triglycérides d'acides gras, des alcools gras, des esters d'acides gras avec des alcools gras et des mélanges de ceux-ci.

5. Formulation selon la revendication 4, dans laquelle le solvant organique approprié pour des produits alimentaires est une graisse qui présente sous la pression normale un point de fusion d'au moins 40 °C.

6. Formulation selon la revendication 5, sous forme d'un granulé.

7. Formulation selon la revendication 4, dans laquelle le solvant organique approprié pour des produits alimentaires est choisi parmi des huiles végétales et animales, qui présentent sous la pression normale un point de fusion inférieur à 40 °C.

8. Formulation selon l'une quelconque des revendications précédentes, contenant encore au moins un autre additif approprié pour des produits alimentaires, qui est choisi parmi des stabilisants vis-à-vis de l'oxydation, des émulsifiants, des adjuvants d'écoulement et des agents destinés à augmenter la viscosité.

9. Procédé pour la préparation d'une formulation selon l'une quelconque des revendications précédentes, comprenant les étapes suivantes :
i) disposition d'une suspension d'un dérivé d'astaxanthine, qui consiste à raison d'au moins 80 % en poids en un composé de formule I selon la revendication 1 ou 2, dans un solvant organique approprié pour des produits alimentaires, qui est choisi parmi des huiles et graisses appropriées pour des produits alimentaires ;
ii) chauffage de la suspension jusqu'à une température d'au moins 100 °C ; et
iii) brusque refroidissement de la solution obtenue dans l'étape ii.

10. Procédé selon la revendication 9, dans lequel la suspension dans l'étape i) est préparée par broyage du dérivé d'astaxanthine dans l'huile, la graisse ou la cire appropriée pour des produits alimentaires.

11. Procédé selon la revendication 9 ou 10, dans lequel les particules du dérivé d'astaxanthine dans la suspension présentent un diamètre moyen en volume de particule, déterminé par diffraction de Fraunhofer, dans la plage de 0,5 à 50 µm.

12. Procédé selon l'une quelconque des revendications 9 à 11, dans lequel on refroidit brusquement la solution, obtenue dans l'étape ii, au moyen d'un courant de gaz froid ou d'un liquide froid, dans lequel le diluant est insoluble.

13. Procédé selon l'une quelconque des revendications 9 à 11, dans lequel on refroidit brusquement la solution, obtenue dans l'étape ii, au moyen d'un courant froid d'un solvant approprié pour des produits alimentaires et/ou au moyen d'un échangeur thermique.

14. Utilisation d'une formulation selon l'une quelconque des revendications 1 à 8, pour la fabrication de préparations d'aliments pour animaux, en particulier de préparations d'aliments solides pour animaux, spécialement de préparations d'aliments sous forme de boulettes pour animaux.

15. Procédé pour la fabrication de préparations d'aliments pour animaux, en particulier d'aliments solides pour animaux, spécialement de boulettes, comprenant :
a) disposition d'une formulation selon l'une quelconque des revendications 1 à 8 ;
b) dilution de la formulation avec une huile liquide appropriée pour des produits alimentaires, avec obtention d'une solution liquide diluée du dérivé d'astaxanthine dans l'huile liquide ; et
c) mélange de la solution liquide obtenue dans l'étape b) avec les composants de la préparation d'aliment pour animaux ou imprégnation d'une préparation d'aliment solide pour animaux avec la solution liquide obtenue dans l'étape b).

16. Procédé selon la revendication 14, dans lequel le solvant est choisi parmi des huiles végétales et animales, qui présentent sous la pression normale un point de fusion inférieur à 40 °C.

17. Procédé selon la revendication 15 ou 16, dans lequel la solution liquide diluée obtenue dans l'étape b) contient le dérivé d'astaxanthine à une concentration de 10 à 1 000 ppm.
